# EUROPÄISCHE PATENTSCHRIFT

(11) **EP 4 161 600 B1**
(45) Veröffentlichungstag und Bekanntmachung des Hinweises auf die Patenterteilung: **17.07.2024**
(21) Anmeldenummer: 21728922.2
(22) Anmeldetag: 28.05.2021
(51) Int. Cl.: A61L 27/04, A61L 27/30, A61F 2/34

(54) **HÜFTIMPLANTATSYSTEM**
HIP IMPLANT SYSTEM
SYSTÈME D'IMPLANT DE HANCHE

(30) Priorität: 03.06.2020 DE 102020206954
(43) Veröffentlichungstag der Anmeldung: 12.04.2023
(73) Patentinhaber: Aesculap AG, 78532 Tuttlingen (DE); IHI Ionbond AG, 8045 Zürich (CH)
(72) Erfinder: BADER, Christian, 78183 Hüfingen (DE); FECHTER, Janine, 72401 Haigerloch (DE); SCHNEIDER, Jens, 78315 Radolfzell (DE)
(74) Vertreter: Patentanwälte Ruff, Wilhelm, Beier, Dauster & Partner mbB
(86) Internationale Anmeldenummer: PCT/EP2021/064374
(87) Internationale Veröffentlichungsnummer: WO 2021/244972

(56) Entgegenhaltungen:
- EP-A1- 0 445 068
- WO-A1-2011/141169
- DE-A1- 2 811 603

## Beschreibung

### ANWENDUNGSGEBIET UND STAND DER TECHNIK

Die Erfindung betrifft ein Hüftimplantatsystem.

Hüftimplantatsysteme kommen heutzutage routinemäßig beim Ersatz von verletzten oder abgenutzten Hüftgelenken zum Einsatz. Entsprechende Hüftimplantatsysteme weisen neben einer künstlichen Hüftpfanne üblicherweise einen direkt in die künstliche Hüftpfanne einsetzbaren künstlichen Hüftpfanneneinsatz, zum Beispiel aus einem Kunststoff, einer Keramik oder einer Metalllegierung, auf. Metallische Hüftpfanneneinsätze bestehen häufig aus einer kobalt- und/oder chromhaltigen Metalllegierung. Zusätzlich können ein direkt in den künstlichen Hüftpfanneneinsatz einsetzbarer künstlicher Hüftgelenkeinsatz und wahlweise ein in den Hüftgelenkeinsatz einsetzbarer künstlicher Gelenkkopf mit oder ohne künstlichen Hüftschaft vorgesehen sein.

Nach der Implantation eines Hüftimplantatsystems kann es aufgrund dynamischer Belastungen häufig zu Mikrobewegungen zwischen der künstlichen Hüftpfanne und dem künstlichen Hüftpfanneneinsatz kommen. Problematisch ist, dass diese Mikrobewegungen eine Reibkorrosion, d.h. ein sogenanntes Fretting, verursachen können, wodurch bei einem künstlichen Hüftpfanneneinsatz aus einer kobalt- und/oder chromhaltigen Metalllegierung Kobalt- und/oder Chromionen in umliegendes Patientengewebe freigesetzt werden können. Überdies können Kobalt- und/oder Chromionen allgemein durch Verschleiß des Hüftpfanneneinsatzes freigesetzt werden. Die in vivo Freisetzung von Kobalt- und/oder Chromionen kann wiederum zu allergischen und/oder toxischen Gewebereaktionen führen, wodurch das Risiko einer chirurgischen Wechseloperation steigt.

EP0445068 A1 offenbart eine künstliche Hüftgelenkpfanne bestehend aus einer metallischen Außenschale mit Verankerungshilfen und aus einer metallischen Innenschale zur Aufnahme eines Gelenkkugelkopfes. Die Außenschale kann aus Titan und die Innenschale aus einer Kobaltlegierung bestehen.

### AUFGABE UND LÖSUNG

Der Erfindung liegt die Aufgabe zugrunde, ein Hüftimplantatsystem bereitzustellen, welches die eingangs im Zusammenhang von gattungsgemäßen Hüftimplantatsystemen beschriebenen Nachteile teilweise oder vollständig vermeidet, insbesondere eine in vivo Freisetzung von Kobalt- und/oder Chromionen reduziert oder sogar vollständig verhindert.

Diese Aufgabe wird erfindungsgemäß gelöst durch ein Hüftimplantatsystem mit den Merkmalen des unabhängigen Anspruchs 1. Bevorzugte Ausgestaltungen des Hüftimplantatsystems sind Gegenstand der abhängigen Ansprüche sowie der Beschreibung. Der Wortlaut sämtlicher Ansprüche wird hiermit durch ausdrückliche Bezugnahme zum Inhalt der Beschreibung gemacht.

Die Erfindung betrifft ein Hüftimplantatsystem.

Das Hüftimplantatsystem weist eine künstliche Hüftpfanne und einen künstlichen Hüftpfanneneinsatz auf. Der künstliche Hüftpfanneneinsatz weist ein Metall oder eine Legierung, vorzugsweise eine kobalt- und/oder chromhaltige Legierung, auf oder besteht aus einem Metall oder einer Legierung, vorzugsweise einer kobalt- und/oder chromhaltigen Legierung. Zweckmäßigerweise ist der künstliche Hüftpfanneneinsatz in die künstliche Hüftpfanne einsetzbar.

Das Hüftimplantatsystem gemäß der vorliegenden Erfindung zeichnet sich besonders dadurch aus, dass der künstliche Hüftpfanneneinsatz wenigstens abschnittsweise, insbesondere nur abschnittsweise oder vollständig, mit einer Keramik-Beschichtung beschichtet ist, wobei die Keramik-Beschichtung Chromnitrid, Chromcarbonitrid und Zirkoniumnitrid aufweist.

Unter dem Ausdruck "Hüftimplantatsystem" soll im Sinne der vorliegenden Erfindung ein Set aus einzelnen Komponenten verstanden werden, wobei die Komponenten, insbesondere während eines chirurgischen Eingriffs, zu einem Hüftimplantat zum Ersatz oder Teilersatz eines natürlichen Hüftgelenks, insbesondere modular, zusammengebaut oder gefügt werden können. Das Set weist als Komponenten wenigstens eine erfindungsgemäße künstliche Hüftpfanne sowie einen erfindungsgemäßen künstlichen Hüftpfanneneinsatz auf. Zusätzlich kann das Set weitere Komponenten, insbesondere wie nachfolgend näher beschrieben, aufweisen.

Unter dem Ausdruck "Keramik-Beschichtung" soll im Sinne der vorliegenden Erfindung eine Beschichtung verstanden werden, welche eine Keramik, insbesondere Hartkeramik, aufweist oder aus einer Keramik, insbesondere Hartkeramik, besteht.

Die Erfindung beruht insbesondere auf der überraschenden Erkenntnis, dass sich eine in vivo Freisetzung von gesundheitsschädigenden Metallionen, insbesondere von Kobalt- und/oder Chromionen, an Berührungsoberflächen modular gefügter Hüftimplantatkomponenten durch eine wenigstens abschnittsweise Beschichtung mit einem keramischen Material (signifikant) reduzieren oder sogar vollständig vermeiden lässt.

Dadurch kann das Risiko postchirurgischer Komplikationen, insbesondere in Form von allergischen oder toxischen Reaktionen, und mithin das Risiko chirurgischer Reinterventionen reduziert oder sogar vollständig eliminiert werden.

Bevorzugt weist die künstliche Hüftpfanne einen wenigstens abschnittsweise, insbesondere nur abschnittsweise oder vollständig, konkav gestalteten Aufnahmebereich zum Aufnehmen des künstlichen Hüftpfanneneinsatzes auf. Der wenigstens abschnittsweise konkav gestaltete Aufnahmebereich weist vorzugsweise eine wenigstens abschnittsweise, insbesondere nur abschnittsweise oder vollständig, konkave Innenfläche der künstlichen Hüftpfanne auf. Insbesondere bevorzugt ist der wenigstens abschnittsweise konkav gestaltete Aufnahmebereich durch die wenigstens abschnittsweise konkave Innenfläche sowie durch eine umlaufende, die wenigstens abschnittsweise konkave Innenfläche begrenzende Kante der künstlichen Hüftpfanne definiert oder gebildet.

Besonders bevorzugt ist die künstliche Hüftpfanne wenigstens abschnittsweise, insbesondere nur abschnittsweise oder vollständig, schalensegmentförmig, insbesondere kugelschalensegmentförmig, gestaltet. Vorzugsweise weist die künstliche Hüftpfanne eine wenigstens abschnittsweise, insbesondere nur abschnittsweise oder vollständig, konvexe Außenfläche und eine wenigstens abschnittsweise, insbesondere nur abschnittsweise oder vollständig, konkave Innenfläche auf, die jeweils durch eine umlaufende Kante der künstlichen Hüftpfanne begrenzt sind.

Alternativ weist die künstliche Hüftpfanne bevorzugt einen wenigstens abschnittsweise, insbesondere nur abschnittsweise oder vollständig, konisch oder kegelstumpfförmig gestalteten Aufnahmebereich zum Aufnehmen des künstlichen Hüftpfanneneinsatzes auf. Der wenigstens abschnittsweise konisch oder kegelstumpfförmig gestaltete Aufnahmebereich weist vorzugsweise eine wenigstens abschnittsweise, insbesondere nur abschnittsweise oder vollständig, konische oder kegelstumpfförmige Innenfläche der künstlichen Hüftpfanne auf. Insbesondere bevorzugt ist der wenigstens abschnittsweise konisch oder kegelstumpfförmig gestaltete Aufnahmebereich durch die wenigstens abschnittsweise konische oder kegelstumpfförmige Innenfläche sowie durch eine umlaufende, die wenigstens abschnittsweise konische oder kegelstumpfförmige Innenfläche begrenzende Kante der künstlichen Hüftpfanne definiert oder gebildet. Vorzugsweise weist die künstliche Hüftpfanne eine wenigstens abschnittsweise, insbesondere nur abschnittsweise oder vollständig, konische oder kegelstumpfförmige Außenfläche und eine wenigstens abschnittsweise, insbesondere nur abschnittsweise oder vollständig, konische oder kegelstumpfförmige Innenfläche auf, die jeweils durch eine umlaufende Kante der künstlichen Hüftpfanne begrenzt sind.

Die künstliche Hüftpfanne weist vorzugsweise ein bioverträgliches Metall auf oder besteht bevorzugt aus einem solchen Metall. Bei dem Metall kann es sich insbesondere um Titan handeln. Ferner kann es bevorzugt sein, dass die künstliche Hüftpfanne an einer Außenfläche, insbesondere an einer der in den vorherigen Absätzen genannten Außenflächen, wenigstens abschnittsweise, insbesondere nur abschnittsweise oder vollständig, mit einer porösen Beschichtung, aufweisend oder bestehend aus Titan und/oder einer Titanlegierung und/oder Hydroxylapatit, beschichtet ist. Dadurch ist vorteilhafterweise eine verbesserte Osteointegration und insbesondere Sekundärstabilität der künstlichen Hüftpfanne erzielbar. Alternativ oder in Kombination kann die Außenfläche der künstlichen Hüftpfanne mit einer Makrostruktur, zum Beispiel mit zahnartigen Vorsprüngen, gestaltet sein. Durch eine entsprechend gestaltete Hüftpfanne lassen sich mit besonderem Vorteil hohe Klemm- sowie Reibungskräfte in vivo realisieren, wodurch eine ausreichende Primärstabilität der künstlichen Hüftpfanne gewährleistet werden kann.

Bevorzugt weist der künstliche Hüftpfanneneinsatz einen wenigstens abschnittsweise, insbesondere nur abschnittsweise oder vollständig, konkav gestalteten Aufnahmebereich zum Aufnehmen eines künstlichen Hüftgelenkeinsatzes auf. Der wenigstens abschnittsweise konkav gestaltete Aufnahmebereich weist vorzugsweise eine wenigstens abschnittsweise, insbesondere nur abschnittsweise oder vollständig, konkave Innenfläche/Gelenk- oder Gleitfläche des künstlichen Hüftpfanneneinsatzes auf. Insbesondere bevorzugt ist der wenigstens abschnittsweise konkav gestaltete Aufnahmebereich durch die wenigstens abschnittsweise konkave Innenfläche/Gelenk- oder Gleitfläche sowie durch eine umlaufende, die wenigstens abschnittsweise konkave Innenfläche/Gelenk- oder Gleitfläche begrenzende Kante des künstlichen Hüftpfanneneinsatzes definiert oder gebildet.

Besonders bevorzugt ist der künstliche Hüftpfanneneinsatz wenigstens abschnittsweise, insbesondere nur abschnittsweise oder vollständig, schalensegmentförmig, insbesondere kugelschalensegmentförmig, gestaltet. Vorzugsweise weist der künstliche Hüftpfanneneinsatz eine wenigstens abschnittsweise, insbesondere nur abschnittsweise oder vollständig, konvexe Außenfläche und eine wenigstens abschnittsweise, insbesondere nur abschnittsweise oder vollständig, konkave Innenfläche/Gelenk- oder Gleitfläche auf, die jeweils durch eine umlaufende Kante des künstlichen Hüftpfanneneinsatzes begrenzt sind.

Besonders bevorzugt weist der künstliche Hüftpfanneneinsatz eine zu dem konkav gestalteten Aufnahmebereich der künstlichen Hüftpfanne komplementäre konvexe Außenform auf.

Alternativ weist der künstliche Hüftpfanneneinsatz bevorzugt einen wenigstens abschnittsweise, insbesondere nur abschnittsweise oder vollständig, konisch oder kegelstumpfförmig gestalteten Aufnahmebereich zum Aufnehmen eines künstlichen Hüftgelenkeinsatzes auf. Der wenigstens abschnittsweise konisch oder kegelstumpfförmig gestaltete Aufnahmebereich weist vorzugsweise eine wenigstens abschnittsweise, insbesondere nur abschnittsweise oder vollständig, konische oder kegelstumpfförmige Innenfläche/Gelenk- oder Gleitfläche des künstlichen Hüftpfanneneinsatzes auf. Insbesondere bevorzugt ist der wenigstens abschnittsweise konisch oder kegelstumpfförmig gestaltete Aufnahmebereich durch die wenigstens abschnittsweise konische oder kegelstumpfförmige Innenfläche/Gelenk- oder Gleitfläche sowie durch eine umlaufende, die wenigstens abschnittsweise konkave Innenfläche/Gelenk- oder Gleitfläche begrenzende Kante des künstlichen Hüftpfanneneinsatzes definiert oder gebildet. Vorzugsweise weist der künstliche Hüftpfanneneinsatz eine wenigstens abschnittsweise, insbesondere nur abschnittsweise oder vollständig, konische oder kegelstumpfförmige Außenfläche und eine wenigstens abschnittsweise, insbesondere nur abschnittsweise oder vollständig, konische oder kegelstumpfförmige Innenfläche/Gelenk- oder Gleitfläche auf, die jeweils durch eine umlaufende Kante des künstlichen Hüftpfanneneinsatzes begrenzt sind. Bevorzugt weist der künstliche Hüftpfanneneinsatz eine zu dem konisch oder kegelstumpfförmig gestalteten Aufnahmebereich der künstlichen Hüftpfanne komplementäre konische oder kegelstumpfförmige Außenform auf.

In weiterer Ausgestaltung der Erfindung ist nur eine Außenfläche, insbesondere nur eine konvexe, konische oder kegelstumpfförmige Außenfläche, des künstlichen Hüftpfanneneinsatzes wenigstens abschnittsweise, insbesondere nur abschnittsweise oder vollständig, mit der Keramik-Beschichtung beschichtet.

In weiterer Ausgestaltung der Erfindung ist nur eine Innenfläche, insbesondere nur eine konkave, konische oder kegelstumpfförmige Innenfläche, des künstlichen Hüftpfanneneinsatzes wenigstens abschnittsweise, insbesondere nur abschnittsweise oder vollständig, mit der Keramik-Beschichtung beschichtet.

In weiterer Ausgestaltung der Erfindung ist der künstliche Hüftpfanneneinsatz vollständig mit der Keramik-Beschichtung beschichtet.

In weiterer Ausgestaltung der Erfindung ist die Keramik-Beschichtung mehrschichtig aufgebaut. Mit anderen Worten ist die Keramik-Beschichtung in weiterer Ausgestaltung der Erfindung als mehrschichtiges Schichtsystem ausgebildet.

In weiterer Ausgestaltung der Erfindung weist die Keramik-Beschichtung wenigstens eine Nichtoxidkeramik auf oder besteht die Keramik-Beschichtung wenigstens aus einer Nichtoxidkeramik. Die wenigstens eine Nichtoxidkeramik ist vorzugsweise ausgewählt aus der Gruppe bestehend aus Chromnitrid (CrN), Chromcarbonitrid (CrCN), Chromzirkoniumnitrid (CrZrN) und Zirkoniumnitrid (ZrN). Bevorzugt weist die Keramik-Beschichtung, insbesondere ausschließlich, Chromnitrid und Chromcarbonitrid auf. Weiter weist die KeramikBeschichtung, insbesondere ausschließlich, Chromnitrid, Chromcarbonitrid und Zirkoniumnitrid auf. Insbesondere kann die Keramik-Beschichtung frei von Chromzirkoniumnitrid sein. Besonders bevorzugt weist die Keramik-Beschichtung, insbesondere ausschließlich, Chromnitrid, Chromcarbonitrid, Chromzirkoniumnitrid und Zirkoniumnitrid auf.

Die Keramik-Beschichtung weist vorzugsweise wenigstens eine Chromnitrid-Schicht (CrN-Schicht), insbesondere (nur) eine Chromnitrid-Schicht, zwei Chromnitrid-Schichten oder drei Chromnitrid-Schichten, auf. Alternativ oder in Kombination weist die Keramik-Beschichtung bevorzugt wenigstens eine Chromcarbonitrid-Schicht (CrCN-Schicht), insbesondere (nur) eine Chromcarbonitrid-Schicht oder zwei Chromcarbonitrid-Schichten, auf. Alternativ oder in Kombination weist die Keramik-Beschichtung bevorzugt wenigstens eine Chromzirkoniumnitrid-Schicht (CrZrN-Schicht), insbesondere (nur) eine Chromzirkoniumnitrid-Schicht, auf. Alternativ oder in Kombination weist die Keramik-Beschichtung bevorzugt wenigstens eine Zirkoniumnitrid-Schicht (ZrN-Schicht), insbesondere (nur) eine Zirkoniumnitrid-Schicht, auf. Insbesondere kann die Keramik-Beschichtung aus einer oder mehreren der vorgenannten Schichten oder aus allen vorgenannten Schichten bestehen.

Unter dem Ausdruck "Chromnitrid-Schicht" soll im Sinne der vorliegenden Erfindung eine Schicht verstanden werden, welche Chromnitrid aufweist, insbesondere als Hauptbestandteil, oder aus Chromnitrid besteht.

Unter dem Ausdruck "Chromcarbonitrid-Schicht" soll im Sinne der vorliegenden Erfindung eine Schicht verstanden werden, welche Chromcarbonitrid aufweist, insbesondere als Hauptbestandteil, oder aus Chromcarbonitrid besteht.

Unter dem Ausdruck "Chromzirkoniumnitrid-Schicht" soll im Sinne der vorliegenden Erfindung eine Schicht verstanden werden, welche Chromzirkoniumnitrid aufweist, insbesondere als Hauptbestandteil, oder aus Chromzirkoniumnitrid besteht.

Unter dem Ausdruck "Zirkoniumnitrid-Schicht" soll im Sinne der vorliegenden Erfindung eine Schicht verstanden werden, welche Zirkoniumnitrid aufweist, insbesondere als Hauptbestandteil, oder aus Zirkoniumnitrid besteht.

Weiter bevorzugt kann die Keramik-Beschichtung eine alternierende Abfolge von/der Chromnitrid-Schichten und Chromcarbonitrid-Schichten aufweisen.

In weiterer Ausgestaltung der Erfindung weist die Keramik-Beschichtung wenigstens eine Chromnitrid-Schicht (CrN-Schicht), insbesondere (nur) eine Chromnitrid-Schicht, zwei Chromnitrid-Schichten oder drei Chromnitrid-Schichten, wenigstens eine Chromcarbonitrid-Schicht (CrCN-Schicht), insbesondere (nur) eine Chromcarbonitrid-Schicht oder zwei Chromcarbonitrid-Schichten, wenigstens eine Chromzirkoniumnitrid-Schicht (CrZrN-Schicht), insbesondere (nur) eine Chromzirkoniumnitrid-Schicht, und wenigstens eine Zirkoniumnitrid-Schicht (ZrN-Schicht), insbesondere (nur) eine Zirkoniumnitrid-Schicht, auf. Insbesondere kann die Keramik-Beschichtung aus den vorgenannten Schichten bestehen.

Alternativ kann die Keramik-Beschichtung wenigstens eine Chromnitrid-Schicht, insbesondere (nur) eine Chromnitrid-Schicht, zwei Chromnitrid-Schichten oder drei Chromnitrid-Schichten, wenigstens eine Chromcarbonitrid-Schicht, insbesondere (nur) eine Chromcarbonitrid-Schicht oder zwei Chromcarbonitrid-Schichten, und wenigstens eine Zirkoniumnitrid-Schicht, insbesondere (nur) eine Zirkoniumnitrid-Schicht, aufweisen. Insbesondere kann die KeramikBeschichtung aus den vorgenannten Schichten bestehen.

In weiterer Ausgestaltung der Erfindung weist die Keramik-Beschichtung drei Chromnitrid-Schichten, zwei Chromcarbonitrid-Schichten, eine, d.h. nur eine, Chromzirkoniumnitrid-Schicht und eine, d.h. nur eine, Zirkoniumnitrid-Schicht auf. Bevorzugt sind die Chromnitrid-Schichten und die Chromcarbonitrid-Schichten in einer alternierenden Abfolge übereinander angeordnet. Insbesondere kann die Keramik-Beschichtung aus den vorgenannten Schichten bestehen.

Alternativ kann die Keramik-Beschichtung drei Chromnitrid-Schichten, zwei Chromcarbonitrid-Schichten und eine, d.h. nur eine, Zirkoniumnitrid-Schicht aufweisen. Bevorzugt sind die Chromnitrid-Schichten und die Chromcarbonitrid-Schichten in einer alternierenden Abfolge übereinander angeordnet. Insbesondere kann die Keramik-Beschichtung aus den vorgenannten Schichten bestehen.

In weiterer Ausgestaltung der Erfindung ist die Keramik-Beschichtung als mehrlagiges oder mehrschichtiges Schichtsystem ausgebildet, bei welchem eine erste Chromnitrid-Schicht, vorzugsweise unmittelbar, von einer ersten Chromcarbonitrid-Schicht, die erste Chromcarbonitrid-Schicht, vorzugsweise unmittelbar, von einer zweiten Chromnitrid-Schicht, die zweite Chromnitrid-Schicht, vorzugsweise unmittelbar, von einer zweiten Chromcarbonitrid-Schicht, die zweite Chromcarbonitrid-Schicht, vorzugsweise unmittelbar, von einer dritten Chromnitrid-Schicht, die dritte Chromnitrid-Schicht, vorzugsweise unmittelbar, von einer Chromzirkoniumnitrid-Schicht und die Chromzirkoniumnitrid-Schicht, vorzugsweise unmittelbar, von einer Zirkoniumnitrid-Schicht bedeckt oder beschichtet ist. Vorzugsweise ist die erste Chromnitrid-Schicht unmittelbar auf einer Oberfläche, insbesondere einer konvexen, konischen oder kegelstumpfförmigen Außenfläche/Gleitfläche und/oder konkaven, konischen oder kegelstumpfförmigen Innenfläche/Gleitfläche, des künstlichen Hüftpfanneneinsatzes ausgebildet.

Alternativ kann die Keramik-Beschichtung als mehrlagiges oder mehrschichtiges Schichtsystem ausgebildet sein, bei welchem eine erste Chromnitrid-Schicht, vorzugsweise unmittelbar, von einer ersten Chromcarbonitrid-Schicht, die erste Chromcarbonitrid-Schicht, vorzugsweise unmittelbar, von einer zweiten Chromnitrid-Schicht, die zweite Chromnitrid-Schicht, vorzugsweise unmittelbar, von einer zweiten Chromcarbonitrid-Schicht, die zweite Chromcarbonitrid-Schicht, vorzugsweise unmittelbar, von einer dritten Chromnitrid-Schicht und die dritte Chromnitrid-Schicht, vorzugsweise unmittelbar, von einer Zirkoniumnitrid-Schicht bedeckt oder beschichtet ist. Vorzugsweise ist die erste Chromnitrid-Schicht unmittelbar auf einer Oberfläche, insbesondere einer konvexen, konischen oder kegelstumpfförmigen Außenfläche/Gleitfläche und/oder konkaven, konischen oder kegelstumpfförmigen Innenfläche/Gleitfläche, des künstlichen Hüftpfanneneinsatzes ausgebildet.

In weiterer Ausgestaltung der Erfindung bildet die Zirkoniumnitrid-Schicht eine äußere Abschluss- oder Deckschicht der Keramik-Beschichtung. Bevorzugt ist die Abschluss- bzw. Deckschicht direkt, d.h. unmittelbar, auf einer Chromnitrid-Schicht, Chromcarbonitrid-Schicht oder Chromzirkoniumnitrid-Schicht ausgebildet.

Die Vorteile der vorliegenden Erfindung sind bei der in den vorherigen Absätzen beschriebenen Nichtoxidkeramik-Beschichtung besonders stark ausgeprägt. Außerdem ist von Vorteil, dass die in den vorherigen Absätzen beschriebene Nichtoxid-Keramikbeschichtung gerade bei einem künstlichen Hüftpfanneneinsatz aus einer kobalt- und/oder chromhaltigen Legierung eine hohe Abriebfestigkeit und mithin eine besonders gute Haftung zeigt.

Alternativ kann die Keramik-Beschichtung wenigstens eine Oxidkeramik aufweisen oder aus wenigstens einer Oxidkeramik bestehen. Die wenigstens eine Oxidkeramik ist vorzugsweise ausgewählt aus der Gruppe bestehend aus Aluminiumoxid (Al₂O₃), Zirkoniumdioxid (ZrO₂), Titandioxid (TiO₂) und Chrom(III)oxid (Cr₂O₃). Bei dem vorgenannten Aluminiumoxid handelt es sich vorzugsweise um Aluminiumoxid des Typs Korund. Bei dem vorgenannten Zirkoniumdioxid handelt es sich vorzugsweise um Zirkoniumdioxid des Typs Baddeleyit. Bei dem vorgenannten Titandioxid handelt es sich vorzugsweise um Titandioxid des Typs Rutil. Bei dem vorgenannten Chrom(III)oxid handelt es sich vorzugsweise um Chrom(III)oxid des Typs Korund.

Die in den vorherigen Absätzen beschriebenen Keramik-Beschichtungen können beispielsweise durch physikalische Gasphasenabscheidung (Physical Vapor Deposition - PVD) auf den künstlichen Hüftpfanneneinsatz aufgebracht werden.

In weiterer Ausgestaltung der Erfindung ist die Keramik-Beschichtung, insbesondere eine der Chromnitrid-Schichten, insbesondere die erste Chromnitrid-Schicht, direkt, d.h. unmittelbar, auf dem künstlichen Hüftpfanneneinsatz, d.h. auf einer Oberfläche, insbesondere einer konvexen, konischen oder kegelstumpfförmigen Außenfläche und/oder konkaven, konischen oder kegelstumpfförmigen Innenfläche/Gelenk- oder Gleitfläche, des künstlichen Hüftpfanneneinsatzes ausgebildet.

Alternativ kann zwischen der Keramik-Beschichtung und dem künstlichen Hüftpfanneneinsatz, d.h. zwischen der Keramik-Beschichtung und einer Oberfläche, insbesondere konvexen, konischen oder kegelstumpfförmigen Außenfläche/Gleitfläche und/oder konkaven, konischen oder kegelstumpfförmigen Innenfläche/Gleitfläche, des künstlichen Hüftpfanneneinsatzes eine Zwischenschicht, insbesondere aus einem Metall, einer Legierung oder einer Keramik, ausgebildet sein. Bei dem Metall kann es sich beispielsweise um Niob, Tantal oder Zirkonium handeln. Bei der Legierung kann es sich beispielsweise um eine Zirkonium-Legierung oder Niob-Legierung handeln. Bei der Keramik kann es sich beispielsweise um eine Keramik handeln, welche Zirkonium, insbesondere in elementarer und/oder metallischer Form, enthält.

In weiterer Ausgestaltung der Erfindung weist die Keramik-Beschichtung eine Dicke von 0,5 µm bis 10 µm, insbesondere 1,5 µm bis 7 µm, bevorzugt 2,5 µm bis 5 µm, besonders bevorzugt 3 µm bis 4 µm, auf. Insbesondere durch die in diesem Absatz offenbarten Beschichtungsdicken lässt sich die Freisetzungsbarriere in vivo für Kobalt- und/oder Chromionen zusätzlich erhöhen, ohne dass ein (nennenswertes) Risiko besteht, dass die Keramik-Beschichtung aufgrund ihrer Schichtdicke von dem künstlichen Hüftpfanneneinsatz abplatzt.

In weiterer Ausgestaltung der Erfindung weist eine Innenfläche, insbesondere eine konkave, konische oder kegelstumpfförmige Innenfläche, des künstlichen Hüftpfanneneinsatzes, insbesondere wie in den vorherigen Absätzen beschrieben, eine Oberflächenrauheit Ra (Mittenrauwert, gemessen nach DIN EN ISO 4287) von ≤ 0,5 µm, bevorzugt ≤ 0,05 µm, und/oder eine Außenfläche, insbesondere konkave, konische oder kegelstumpfförmige Außenfläche, des künstlichen Hüftpfanneneinsatzes, insbesondere wie in den vorherigen Absätzen beschrieben, eine Oberflächenrauheit Ra (Mittenrauwert, gemessen nach DIN EN ISO 4287) von ≤ 1 µm, bevorzugt ≤ 0,6 µm, auf. Insbesondere durch die in diesem Absatz offenbarten Oberflächenrauheiten ist mit besonderem Vorteil eine Verzahnung von Oberflächen des künstlichen Hüftpfanneneinsatzes und der künstlichen Hüftpfanne und/oder eine Verzahnung von Oberflächen des künstlichen Hüftpfanneneinsatzes und eines künstlichen Hüftgelenkeinsatzes erzielbar. Ferner haben insbesondere die in diesem Absatz offenbarten Oberflächenrauheiten den Vorteil, dass sie das Risiko eines Abplatzens der KeramikBeschichtung von dem künstlichen Hüftpfanneneinsatz reduzieren und obendrein - im Falle von Mikrobewegungen des künstlichen Hüftpfanneneinsatzes und/oder der künstlichen Hüftpfanne und/oder des künstlichen Hüftgelenkeinsatzes weniger Abrieb verursachen.

In weiterer Ausgestaltung der Erfindung weist der künstliche Hüftpfanneneinsatz, insbesondere im nicht beschichteten Zustand, d.h. ohne die Keramik-Beschichtung, eine Dicke, insbesondere Wanddicke, von 2 mm bis 3 mm auf. Die in diesem Absatz offenbarten Dicken, insbesondere Wanddicken, haben den Vorteil, dass sie eine flexible, insbesondere elastische, Gestaltung des künstlichen Hüftpfanneneinsatzes erlauben. Obendrein ermöglichen sie in vorteilhafter Weise nach einem Fügen des künstlichen Hüftpfanneneinsatzes in die künstliche Hüftpfanne höhere Kontaktspannungen, wodurch das Risiko von Mikrobewegungen des künstlichen Hüftpfanneneinsatzes und/oder der künstlichen Hüftpfanne und mithin das Risiko einer Reibkorrosion zusätzlich vermindert werden kann.

In weiterer Ausgestaltung der Erfindung weist der künstliche Hüftpfanneneinsatz einen Konuswinkel θ von 15° bis 25°, insbesondere 16° bis 21°, auf. Unter dem Begriff "Konuswinkel" soll hierbei der Winkel zwischen einer Symmetrieachse (Zentralachse) und einer konischen Außenfläche des Hüftpfanneneinsatzes verstanden werden. Insbesondere durch die in diesem Absatz offenbarten Konuswinkel lassen sich hohe Klemmkräfte zwischen dem künstlichen Hüftpfanneneinsatz und der künstlichen Hüftpfanne realisieren, wodurch sich das Risiko von Relativbewegungen zwischen dem künstlichen Hüftpfanneneinsatz und der künstlichen Hüftpfanne und somit das Risiko einer unerwünschten Metallionenfreisetzung, insbesondere Kobalt- und/oder Chromionenfreisetzung, ebenfalls reduzieren lässt.

In weiterer Ausgestaltung der Erfindung sind der künstliche Hüftpfanneneinsatz und die künstliche Hüftpfanne in einem zusammengebauten oder gefügten Zustand miteinander kraftoder reibschlüssig verbunden. Vorzugsweise sind der künstliche Hüftpfanneneinsatz und die künstliche Hüftpfanne in einem zusammengebauten oder gefügten Zustand miteinander klemmverbunden, vorzugsweise konisch klemmverbunden. Besonders bevorzugt sind der künstliche Hüftpfanneneinsatz und die künstliche Hüftpfanne in einem zusammengebauten oder gefügten Zustand über eine konische oder kegelstumpfförmige Außenfläche des künstlichen Hüftpfanneneinsatzes und eine hierzu komplementäre konische oder kegelstumpfförmige Innenfläche der künstlichen Hüftpfanne miteinander klemmverbunden.

In weiterer Ausgestaltung der Erfindung handelt es sich bei der Legierung um eine kobaltund/oder chromhaltigen Legierung, insbesondere um eine Kobalt-Chrom-Molybdän-Legierung. Die Kobalt-Chrom-Molybdän-Legierung kann einen Kobaltmassenanteil von 62 % bis 66 %, einen Chrommassenanteil von 27 % bis 31 % und einen Molybdänmassenanteil von 4 % bis 5 % aufweisen. Ferner kann die Kobalt-Chrom-Molybdän-Legierung, insbesondere in kleinen Mengen und/oder in Form von Verunreinigungen, Kohlenstoff und/oder Silizium und/oder Mangan und/oder Eisen aufweisen.

In weiterer Ausgestaltung der Erfindung weist das Hüftimplantatsystem ferner einen künstlichen Hüftgelenkeinsatz auf. Der künstliche Hüftgelenkeinsatz ist zweckmäßigerweise in den künstlichen Hüftpfanneneinsatz einsetzbar. Bevorzugt bilden der künstliche Hüftpfanneneinsatz und der künstliche Hüftgelenkeinsatz in einem gefügten oder montierten Zustand, insbesondere nach Einsetzen des künstlichen Hüftpfanneneinsatzes in den künstlichen Hüftgelenkeinsatz, ein Kugelgelenk aus.

Der künstliche Hüftgelenkeinsatz weist vorzugsweise einen wenigstens abschnittsweise, insbesondere nur abschnittsweise oder vollständig, konkav gestalteten Aufnahmebereich zum Aufnehmen eines künstlichen Hüftgelenkkopfes auf. Der wenigstens abschnittsweise konkav gestaltete Aufnahmebereich weist vorzugsweise eine wenigstens abschnittsweise, insbesondere nur abschnittsweise oder vollständig, konkave Innenfläche/Gelenk- oder Gleitfläche des künstlichen Hüftgelenkeinsatzes auf. Insbesondere bevorzugt ist der wenigstens abschnittsweise konkav gestaltete Aufnahmebereich durch die wenigstens abschnittsweise konkave Innenfläche/Gelenk- oder Gleitfläche sowie durch eine umlaufende, die wenigstens abschnittsweise konkave Innenfläche/Gelenk- oder Gleitfläche begrenzende Kante des künstlichen Hüftgelenkeinsatzes definiert oder gebildet.

Der künstliche Hüftgelenkeinsatz ist vorzugsweise wenigstens abschnittsweise, insbesondere nur abschnittsweise oder vollständig, schalensegmentförmig, insbesondere kugelschalensegmentförmig, gestaltet. Vorzugsweise weist der künstliche Hüftgelenkeinsatz eine wenigstens abschnittsweise, insbesondere nur abschnittsweise oder vollständig, konvexe Außenfläche/Gelenk- oder Gleitfläche und eine wenigstens abschnittsweise, insbesondere nur abschnittsweise oder vollständig, konkave Innenfläche/Gelenk- oder Gleitfläche auf, die jeweils durch eine umlaufende Kante des künstlichen Hüftgelenkeinsatzes begrenzt sind. Besonders bevorzugt weist der künstliche Hüftgelenkeinsatz eine zu dem konkav gestalteten Aufnahmebereich des künstlichen Hüftpfanneneinsatzes komplementäre konvexe Außenform auf.

Alternativ weist der künstliche Hüftgelenkeinsatz vorzugsweise einen wenigstens abschnittsweise, insbesondere nur abschnittsweise oder vollständig, konisch oder kegelstumpfförmig gestalteten Aufnahmebereich zum Aufnehmen eines künstlichen Hüftgelenkkopfs auf. Der wenigstens abschnittsweise konisch oder kegelstumpfförmig gestaltete Aufnahmebereich weist vorzugsweise eine wenigstens abschnittsweise, insbesondere nur abschnittsweise oder vollständig, konische oder kegelstumpfförmige Innenfläche/Gelenk- oder Gleitfläche des künstlichen Hüftgelenkeinsatzes auf. Insbesondere bevorzugt ist der wenigstens abschnittsweise konisch oder kegelstumpfförmig gestaltete Aufnahmebereich durch die wenigstens abschnittsweise konische oder kegelstumpfförmige Innenfläche/Gelenk- oder Gleitfläche sowie durch eine umlaufende, die wenigstens abschnittsweise konkave Innenfläche/Gelenk- oder Gleitfläche begrenzende Kante des künstlichen Hüftgelenkeinsatzes definiert oder gebildet. Vorzugsweise weist der künstliche Hüftgelenkeinsatz eine wenigstens abschnittsweise, insbesondere nur abschnittsweise oder vollständig, konische oder kegelstumpfförmige Außenfläche/Gelenk- oder Gleitfläche und eine wenigstens abschnittsweise, insbesondere nur abschnittsweise oder vollständig, konische oder kegelstumpfförmige Innenfläche/Gelenk- oder Gleitfläche auf, die jeweils durch eine umlaufende Kante des künstlichen Hüftgelenkeinsatzes begrenzt sind. Besonders bevorzugt weist der künstliche Hüftgelenkeinsatz eine zu dem konisch oder kegelstumpfförmig gestalteten Aufnahmebereich des künstlichen Hüftpfanneneinsatzes komplementäre konische oder kegelstumpfförmige Außenform auf.

Vorzugsweise weist der künstliche Hüftgelenkeinsatz, insbesondere ausschließlich, einen Kunststoff, bevorzugt Polyethylen, insbesondere ultrahochmolekulares Polyethylen, und optional ein Antioxidans, insbesondere Vitamin E, auf.

In weiterer Ausgestaltung der Erfindung weist das Hüftimplantatsystem ferner einen künstlichen Hüftgelenkkopf, insbesondere mit oder ohne einem künstlichen Hüftschaft, auf. Zweckmäßigerweise ist der künstliche Hüftgelenkkopf in den künstlichen Hüftgelenkeinsatz einsetzbar. Bevorzugt bilden der künstliche Hüftgelenkeinsatz und der künstliche Hüftgelenkkopf in einem gefügten oder montierten Zustand, insbesondere nach Einsetzen des künstlichen Hüftgelenkkopfes in den künstlichen Hüftgelenkeinsatz, (ebenfalls) ein Kugelgelenk aus. Vorzugsweise weist der künstliche Hüftgelenkeinsatz eine Einsatzöffnung auf, die kleiner ist als die Außenabmessung des (einzusetzenden) künstlichen Hüftgelenkkopfes. Anders ausgedrückt ist die Einsatzöffnung des künstlichen Hüftgelenkeinsatzes vorzugsweise kleiner als der größte Abstand zwischen zwei Punkten auf einer Außenfläche/Gleitfläche, insbesondere konvexen, konischen oder kegelstumpfförmigen Außenfläche/Gleitfläche, des (einzusetzenden) künstlichen Hüftgelenkkopfes. Bevorzugt bilden daher der künstliche Hüftgelenkeinsatz und der künstliche Hüftgelenkkopf in einem gefügten oder montierten Zustand, insbesondere nach Einsetzen des künstlichen Hüftgelenkkopfes in den künstlichen Hüftgelenkeinsatz, ein Nussgelenk aus.

Der künstliche Hüftgelenkkopf ist vorzugsweise kugelförmig oder teilkugelförmig gestaltet. Bevorzugt weist der künstliche Hüftgelenkkopf einen Aufnahmebereich, insbesondere in Form einer Aufnahmeaussparung oder -vertiefung, bevorzugt in Form einer zylinder- oder konusförmigen Aufnahmeaussparung oder -vertiefung, zum Aufnehmen eines zylinder- oder konusförmigen Abschnittes eines künstlichen Hüftschaftes auf. Der Aufnahmebereich kann einen kreisförmigen oder nicht kreisförmigen, insbesondere polygonen, wie beispielsweise viereckigen, Querschnitt aufweisen.

Alternativ kann der künstliche Hüftgelenkkopf wenigstens abschnittsweise, insbesondere nur abschnittsweise oder vollständig, schalensegmentförmig, insbesondere kugelschalensegmentförmig, gestaltet sein. Vorzugsweise weist der künstliche Hüftgelenkkopf eine wenigstens abschnittsweise, insbesondere nur abschnittsweise oder vollständig, konvexe Außenfläche/Gelenk- oder Gleitfläche und eine wenigstens abschnittsweise, insbesondere nur abschnittsweise oder vollständig, konkave Innenfläche auf, die jeweils durch eine umlaufende Kante des künstlichen Hüftgelenkkopfes begrenzt sind. Besonders bevorzugt weist der künstliche Hüftgelenkkopf eine zu dem konkav gestalteten Aufnahmebereich des künstlichen Hüftgelenkeinsatzes komplementäre konvexe Außenform auf. Alternativ weist der künstliche Hüftgelenkkopf vorzugsweise eine zu dem konisch oder kegelstumpfförmig gestalteten Aufnahmebereich des künstlichen Hüftgelenkeinsatzes komplementäre konische oder kegelstumpfförmige Außenform auf.

Ferner kann der Hüftgelenkkopf eine Keramik oder einen Kunststoff aufweisen oder aus einer Keramik oder einem Kunststoff bestehen.

Weitere Merkmale und Vorteile der Erfindung ergeben sich aus den Ansprüchen sowie aus der nachfolgenden Beschreibung bevorzugter Ausführungsbeispiele der Erfindung, die anhand der Zeichnungen dargestellt sind.

### FIGURENKURZBESCHREIBUNGEN

- Fig. 1: zeigt eine schematische Explosionsdarstellung einer Ausführungsform eines erfindungsgemäßen Hüftimplantatsystems,
- Fig. 2: zeigt schematisch eine vergrößerte Detaildarstellung einer Keramik-Beschichtung eines erfindungsgemäßen Hüftimplantatsystems in einem Bereich A nach Fig. 1 und
- Fig. 3: zeigt schematisch eine Ausführungsform eines erfindungsgemäßen Hüftimplantatsystems im montierten oder implantierten Zustand.

### AUSFÜHRLICHE FIGURENBESCHREIBUNGEN

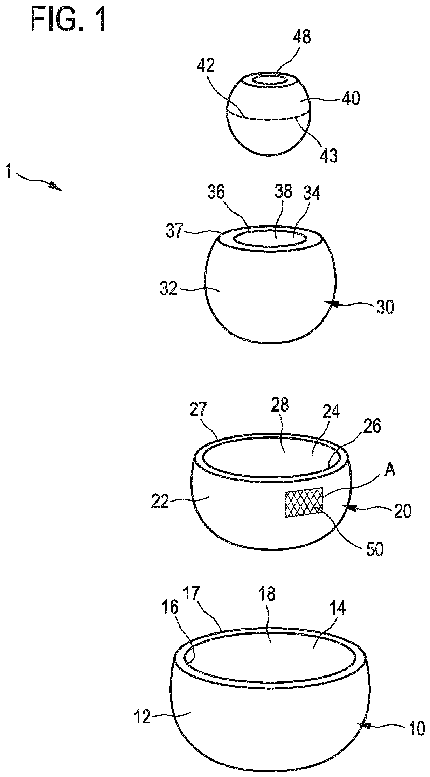

Fig. 1 zeigt schematisch eine Ausführungsform eines Hüftimplantatsystems 1 gemäß der vorliegenden Erfindung.

Das Hüftimplantatsystem 1 weist eine künstliche Hüftpfanne 10 sowie einen in die künstliche Hüftpfanne 10 einsetzbaren künstlichen Hüftpfanneneinsatz 20 auf. Ferner kann das Hüftimplantatsystem 1 einen künstlichen Hüftgelenkeinsatz 30 und insbesondere einen künstlichen Hüftgelenkkopf 40 aufweisen.

Die künstliche Hüftpfanne 10 ist vorzugsweise wenigstens abschnittsweise, insbesondere nur abschnittsweise oder vollständig, kugelschalensegmentförmig gestaltet. Bevorzugt weist die künstliche Hüftpfanne 10 eine wenigstens abschnittsweise konvexe Außenfläche 12 und eine wenigstens abschnittsweise konkave Innenfläche 14 auf, die jeweils durch eine umlaufende Kante 16, 17 der künstlichen Hüftpfanne 10 begrenzt sind. Dabei bilden die wenigstens abschnittsweise konkave Innenfläche 14 und die umlaufende Kante 16 einen wenigstens abschnittsweise konkav gestalteten Aufnahmebereich 18 für den künstlichen Hüftpfanneneinsatz 20.

Die künstliche Hüftpfanne 10 ist vorzugsweise aus einem bioverträglichen Metall, wie beispielsweise Titan, gefertigt. Zur Erzielung einer ausreichenden Osteointegration und insbesondere Sekundärstabilität kann auf der wenigstens abschnittsweise konvex gestalteten Außenseite der künstlichen Hüftpfanne 10 eine offenporige Titan- oder Hydroxylapatit-Beschichtung ausgebildet sein.

Der künstliche Hüftpfanneneinsatz 20 ist ebenfalls bevorzugt wenigstens abschnittsweise, insbesondere nur abschnittsweise oder vollständig, kugelschalensegmentförmig gestaltet. Vorzugsweise weist der künstliche Hüftpfanneneinsatz 20 eine wenigstens abschnittsweise konvexe Außenfläche 22 und eine wenigstens abschnittsweise konkave Innen- oder Gleitfläche 24 auf, die jeweils durch eine umlaufende Kante 26, 27 des künstlichen Hüftpfanneneinsatzes 20 begrenzt sind. Dabei bilden die wenigstens abschnittsweise konkave Innen- oder Gleitfläche 24 und die umlaufende Kante 26 einen wenigstens abschnittsweise konkav gestalteten Aufnahmebereich 28 für den künstlichen Hüftgelenkeinsatz 30.

Die wenigstens abschnittsweise konvexe Außenfläche 22 des künstlichen Hüftpfanneneinsatzes 20 weist vorzugsweise eine Oberflächenrauheit Ra von ≤ 1 µm, bevorzugt ≤ 0,6 µm, auf. Dadurch ist in vorteilhafter Weise eine Optimierung der Verzahnung der wenigstens abschnittsweise konvexen Außenfläche 22 mit der wenigstens abschnittsweise konkaven Innenfläche 14 der künstlichen Hüftpfanne 10 erzielbar.

Die wenigstens abschnittsweise konkave Innen- oder Gleitfläche 24 des künstlichen Hüftpfanneneinsatzes 20 weist vorzugsweise eine Oberflächenrauheit Ra von ≤ 0,5 µm, bevorzugt ≤ 0,05 µm, auf. Dadurch ist mit besonderem Vorteil eine optimierte Verzahnung der wenigstens abschnittsweise konkaven Innen- oder Gleitfläche 24 mit einer Außen- oder Gleitfläche des künstlichen Hüftgelenkeinsatzes 30 erzielbar.

Ferner sind der künstliche Hüftpfanneneinsatz 20 und die künstliche Hüftpfanne 10 in einem montierten Zustand miteinander klemmverbunden, vorzugsweise konisch klemmverbunden.

Der künstliche Hüftpfanneneinsatz 20 besteht aus einer kobalt- und/oder chromhaltigen Legierung, vorzugsweise aus einer Kobalt-Chrom-Molybdän-Legierung.

Der künstliche Hüftpfanneneinsatz 20 ist wenigstens abschnittsweise, insbesondere nur abschnittsweise oder vollständig, mit einer Keramik-Beschichtung 50 beschichtet oder versehen. Die Keramikbeschichtung weist vorzugsweise Chromnitrid (CrN), Chromcarbonitrid (CrCN), Chromzirkoniumnitrid (CrZrN) und Zirkoniumnitrid (ZrN) auf. Insbesondere kann die Keramik-Beschichtung 50 aus den vorgenannten Keramikmaterialien bestehen.

Insbesondere bevorzugt weist die Keramik-Beschichtung 50 wenigstens eine Chromnitrid-Schicht (CrN-Schicht), insbesondere (nur) eine oder mehrere Chromnitrid-Schichten, wie beispielsweise zwei oder drei Chromnitrid-Schichten, und/oder wenigstens eine Chromcarbonitrid-Schicht (CrCN-Schicht), insbesondere (nur) eine oder mehrere Chromcarbonitrid-Schichten, wie beispielsweise zwei Chromcarbonnitrid-Schichten, und/oder wenigstens eine Chromzirkoniumnitrid-Schicht (CrZrN-Schicht), insbesondere (nur) eine oder mehrere Chromzirkoniumnitrid-Schichten, und/oder wenigstens eine Zirkoniumnitrid-Schicht (ZrN-Schicht), insbesondere (nur) eine oder mehrere Zirkoniumnitrid-Schichten, auf. Die Keramik-Beschichtung 50 kann insbesondere aus einer oder mehreren der vorgenannten Schichten oder aus allen vorgenannten Schichten bestehen.

Bevorzugt weist die Keramik-Beschichtung 50 eine Dicke von 0,5 µm bis 10 µm auf.

In einem nicht beschichteten Zustand kann der künstliche Hüftpfanneneinsatz 20 ferner insbesondere eine Wanddicke von 2 mm bis 3 mm aufweisen.

Durch die Keramik-Beschichtung 50 kann in vorteilhafter Weise eine in vivo Freisetzung von Kobalt- und/oder Chromionen in umliegendes Patientengewebe reduziert oder sogar vollständig vermieden werden. Dies wiederum reduziert das Risiko von postchirurgischen Komplikationen, insbesondere in Form von Infektionen. Dadurch kann im Ergebnis das Risiko einer postchirurgischen Reintervention signifikant reduziert werden.

Der künstliche Hüftgelenkeinsatz 30 ist ebenfalls vorzugsweise wenigstens abschnittsweise, insbesondere nur abschnittsweise oder vollständig, kugelschalensegmentförmig gestaltet. Bevorzugt besitzt der künstliche Hüftgelenkeinsatz 30 eine wenigstens abschnittsweise konvexe Außen- oder Gleitfläche 32 und eine wenigstens abschnittsweise konkave Innen- oder Gleitfläche 34, die jeweils durch eine umlaufende Kante 36, 37 des künstlichen Hüftgelenkeinsatzes 30 begrenzt sind. Dabei bilden die wenigstens abschnittsweise konkave Innen- oder Gleitfläche 34 und die umlaufende Kante 36 einen wenigstens abschnittsweise konkav gestalteten Aufnahmebereich 38 für den künstlichen Hüftgelenkkopf 40.

Bevorzugt bilden die wenigstens abschnittsweise konkave Innen- oder Gleitfläche 24 des künstlichen Hüftpfanneneinsatzes 20 und die wenigstens abschnittsweise konvexe Außen- oder Gleitfläche 32 des künstlichen Hüftgelenkeinsatzes 30 nach Implantation des Hüftimplantatsystems 1 ein Kugelgelenk aus.

Der künstliche Hüftgelenkeinsatz 30 besteht aus einem Kunststoff, vorzugsweise aus Polyethylen, insbesondere ultrahochmolekularem Polyethylen, und wahlweise aus einem Antioxidans. Bei dem Antioxidans kann es sich zum Beispiel um Vitamin E handeln.

Der künstliche Hüftgelenkkopf 40 ist vorzugsweise kugel- oder teilkugelförmig oder (ebenfalls) wenigstens abschnittsweise kugelschalensegmentförmig gestaltet. Der künstliche Hüftgelenkkopf 40 weist einen Aufnahmebereich 48 zur Aufnahme eines künstlichen Hüftschafts, insbesondere zur Aufnahme eines vorzugsweise zylinder- oder konusförmigen Abschnitts eines künstlichen Hüftschafts, auf. Der Aufnahmebereich 48 ist vorzugsweise als Vertiefung mit einem kreisförmigen oder nicht kreisförmigen Querschnitt gestaltet. Beispielsweise kann die Vertiefung einen rechteckigen Querschnitt aufweisen.

Bevorzugt bilden die wenigstens abschnittsweise konkave Innen- oder Gleitfläche 34 des künstlichen Hüftgelenkeinsatzes 30 und eine wenigstens abschnittsweise konvexe Außen- oder Gleitfläche 42 des künstlichen Hüftgelenkkopfs 40 ein sogenanntes Nussgelenk. Ein solches Nussgelenk zeichnet sich dadurch aus, dass die wenigstens abschnittsweise konvex gestaltete Außen- oder Gleitfläche 42 des künstlichen Hüftgelenkkopfs 40 über seinen Äquator 43 hinaus von der wenigstens abschnittsweise konkav gestalteten Innen- oder Gleitfläche 34 des künstlichen Hüftgelenkeinsatzes 30 eingefasst ist, so dass einem Auseinanderfallen dieser Komponenten des Hüftimplantatsystems 1 vorgebeugt ist.

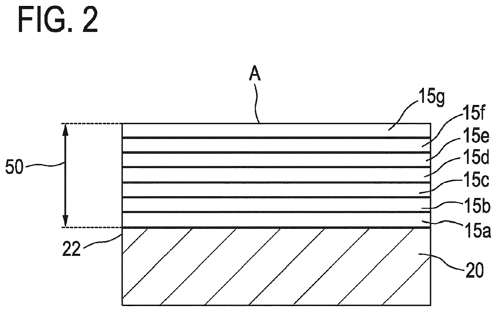

Fig. 2 zeigt schematisch eine vergrößerte Detaildarstellung einer erfindungsgemäß bevorzugten Keramik-Beschichtung 50 in einem Bereich A auf der wenigstens abschnittsweise konvexen Außenfläche 22 des künstlichen Hüfteinsatzes 20 nach Fig. 1.

Die Keramik-Beschichtung 50 ist als mehrschichtiges Schichtsystem mit nachfolgender Schichtabfolge auf der wenigstens abschnittsweise konvexen Außenfläche 22 des künstlichen Hüfteinsatzes 20 ausgebildet:
Die wenigstens abschnittsweise konvexe Außenfläche 22 wird unmittelbar von einer ersten Chromnitrid-Schicht 15a bedeckt. Die erste Chromnitrid-Schicht 15a wird unmittelbar von einer ersten Chromcarbonitrid-Schicht 15b bedeckt. Die erste Chromcarbonitrid-Schicht 15b wird unmittelbar von einer zweiten Chromnitrid-Schicht 15c bedeckt. Die zweite Chromnitrid-Schicht 15c wird unmittelbar von einer zweiten Chromcarbonitrid-Schicht 15d bedeckt. Die zweite Chromcarbonitrid-Schicht 15d wird unmittelbar von einer dritten Chromnitrid-Schicht 15e bedeckt. Die dritte Chromnitrid-Schicht 15e wird unmittelbar von einer Chromzirkoniumnitrid-Schicht 15f bedeckt. Die Chromzirkoniumnitrid-Schicht 15f wird unmittelbar von einer Zirkoniumnitrid-Schicht 15g, die gleichzeitig die Deckschicht der Keramik-Beschichtung 50 darstellt, bedeckt. Alternativ kann die dritte Chromnitrid-Schicht 15e unmittelbar von einer Deckschicht aus Zirkoniumnitrid bedeckt sein (nicht dargestellt).

Alternativ kann zwischen dem künstlichen Hüftpfanneneinsatz und der Keramik-Beschichtung 50 eine Zwischenschicht, beispielsweise aus Zirkonium, einer Zirkonium-Legierung oder einer zirkoniumhaltigen Keramik, ausgebildet sein (nicht dargestellt).

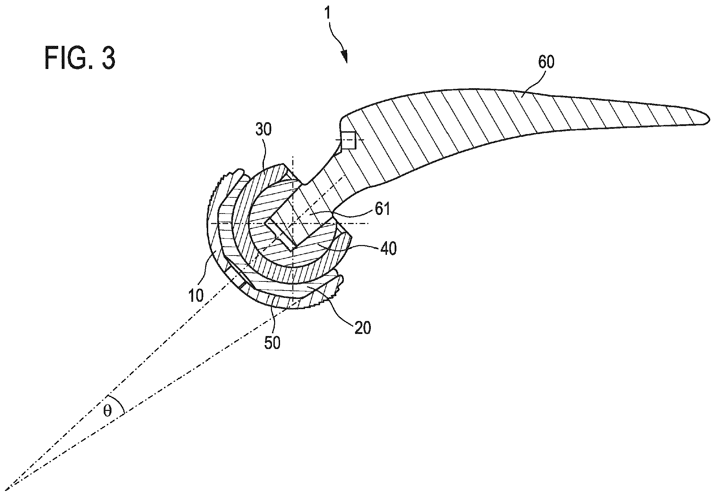

Fig. 3 zeigt eine Ausführungsform eines Hüftimplantatsystem 1 in einem montierten oder implantierten Zustand.

Das Hüftimplantatsystem weist eine künstliche Hüftpfanne 10, einen künstlichen Hüftpfanneneinsatz 20, einen künstlichen Hüftgelenkeinsatz 30, einen künstlichen Hüftgelenkkopf 40 sowie einen künstliche Hüftschaft 60 auf.

Der künstliche Hüftpfanneneinsatz 20 ist wenigstens abschnittsweise, insbesondere nur abschnittsweise oder vollständig, mit einer Keramik-Beschichtung 50 beschichtet. Der künstliche Hüftpfanneneinsatz 20 kann insbesondere einen Konuswinkel θ von 15° bis 25°, insbesondere 16° bis 21°, aufweisen. Dadurch sind im gefügten Zustand in vorteilhafterweise hohe Klemmkräfte zwischen dem künstlichen Hüftpfanneneinsatz 20 und der künstlichen Hüftpfanne 10 realisierbar.

Der künstliche Hüftschaft 60 ist über einen zylinder- oder konusförmigen Abschnitt 61 in den künstlichen Hüftgelenkkopf 40 eingesetzt.

Bezüglich weiterer Merkmale und Vorteile des Hüftimplantatsystems 1, insbesondere der künstlichen Hüftpfanne 10, des künstlichen Hüftpfanneneinsatzes 20, des künstlichen Hüftgelenkeinsatzes 30, des künstlichen Hüftgelenkkopfs 40, der Keramik-Beschichtung 50 sowie des künstlichen Hüftschafts 60, wird vollständig auf die Beschreibungen zur Fig. 1 und 2 Bezug genommen. Die dort insoweit beschriebenen Merkmale und Vorteile gelten sinngemäß auch für das in Fig. 3 dargestellte Hüftimplantatsystem 1.

## Patentansprüche

1. Hüftimplantatsystem (1), aufweisend
- eine künstliche Hüftpfanne (10) und
- einen künstlichen Hüftpfanneneinsatz (20), aufweisend oder bestehend aus einem Metall oder einer Legierung,
wobei der künstliche Hüftpfanneneinsatz (20) wenigstens abschnittsweise mit einer Keramik-Beschichtung (50) beschichtet ist, **dadurch gekennzeichnet, dass** die KeramikBeschichtung Chromnitrid, Chromcarbonitrid und Zirkoniumnitrid aufweist.

2. Hüftimplantatsystem (1) nach Anspruch 1, **dadurch gekennzeichnet, dass** nur eine Außenfläche (22), insbesondere nur eine konvexe, konische oder kegelstumpfförmige Außenfläche, des künstlichen Hüftpfanneneinsatzes (20) oder nur eine Innenfläche (24), insbesondere nur eine konkave, konische oder kegelstumpfförmige Innenfläche, des künstlichen Hüftpfanneneinsatzes (20) wenigstens abschnittsweise mit der KeramikBeschichtung (50) beschichtet ist.

3. Hüftimplantatsystem (1) nach Anspruch 1, **dadurch gekennzeichnet, dass** der künstliche Hüftpfanneneinsatz (20) vollständig mit der Keramik-Beschichtung (50) beschichtet ist.

4. Hüftimplantatsystem (1) nach einem der vorhergehenden Ansprüche, **dadurch gekennzeichnet, dass** die Keramik-Beschichtung (50) mehrschichtig aufgebaut ist.

5. Hüftimplantatsystem (1) nach einem der vorhergehenden Ansprüche, **dadurch gekennzeichnet, dass** die Keramik-Beschichtung (50) wenigstens eine Chromnitrid-Schicht, wenigstens eine Chromcarbonitrid-Schicht, wenigstens eine Chromzirkoniumnitrid-Schicht und wenigstens eine Zirkoniumnitrid-Schicht aufweist.

6. Hüftimplantatsystem (1) nach einem der vorhergehenden Ansprüche, **dadurch gekennzeichnet, dass** die Keramik-Beschichtung (50) drei Chromnitrid-Schichten, zwei Chromcarbonitrid-Schichten, eine Chromzirkoniumnitrid-Schicht und eine Zirkoniumnitrid-Schicht aufweist oder aus den vorgenannten Schichten besteht.

7. Hüftimplantatsystem (1) nach einem der vorhergehenden Ansprüche, **dadurch gekennzeichnet, dass** die Keramik-Beschichtung (50) als mehrschichtiges Schichtsystem ausgebildet, bei welchem eine erste Chromnitrid-Schicht (15a) von einer ersten Chromcarbonitrid-Schicht (15b), die erste Chromcarbonitrid-Schicht (15b) von einer zweiten Chromnitrid-Schicht (15c), die zweite Chromnitrid-Schicht (15c) von einer zweiten Chromcarbonitrid-Schicht (15d), die zweite Chromcarbonitrid-Schicht (15d) von einer dritten Chromnitrid-Schicht (15e), die dritte Chromnitrid-Schicht (15e) von einer Chromzirkoniumnitrid-Schicht (15f) und die Chromzirkoniumnitrid-Schicht (15f) von einer Zirkoniumnitrid-Schicht (15g) bedeckt ist.

8. Hüftimplantatsystem (1) nach Anspruch 6 oder 7, **dadurch gekennzeichnet, dass** die Zirkoniumnitrid-Schicht (15g) eine äußere Deckschicht der Keramik-Beschichtung bildet.

9. Hüftimplantatsystem (1) nach einem der vorhergehenden Ansprüche, insbesondere nach Anspruch 6 oder 7, **dadurch gekennzeichnet, dass** die Keramik-Beschichtung (50), insbesondere eine der Chromnitrid-Schichten oder die erste Chromnitrid-Schicht (15a), unmittelbar auf dem künstlichen Hüftpfanneneinsatz (20) ausgebildet ist.

10. Hüftimplantatsystem (1) nach einem der vorhergehenden Ansprüche, **dadurch gekennzeichnet, dass** die Keramik-Beschichtung (50) eine Dicke von 0,5 µm bis 10 µm, insbesondere 1,5 µm bis 7 µm, bevorzugt 2,5 µm bis 5 µm, aufweist.

11. Hüftimplantatsystem (1) nach einem der vorhergehenden Ansprüche, **dadurch gekennzeichnet, dass**
- eine Innenfläche des künstlichen Hüftpfanneneinsatzes (20) eine Oberflächenrauheit von ≤ 0,5 µm, bevorzugt ≤ 0,05 µm, und/oder
- eine Außenfläche des künstlichen Hüftpfanneneinsatzes (20) eine Oberflächenrauheit von ≤ 1 µm, bevorzugt ≤ 0,6 µm, und/oder
- der künstliche Hüftpfanneneinsatz (20) eine Wanddicke von 2 mm bis 3 mm und/oder
- der künstliche Hüftpfanneneinsatz (20) einen Konuswinkel θ von 15° bis 25°, insbesondere 16° bis 21°, aufweist.

12. Hüftimplantatsystem (1) nach einem der vorhergehenden Ansprüche, **dadurch gekennzeichnet, dass** der künstliche Hüftpfanneneinsatz (20) und die künstliche Hüftpfanne (10) in einem gefügten Zustand miteinander reibschlüssig verbunden, insbesondere miteinander konisch klemmverbunden, sind.

13. Hüftimplantatsystem (1) nach einem der vorhergehenden Ansprüche, **dadurch gekennzeichnet, dass** es sich bei der Legierung um eine kobalt- und/oder chromhaltige Legierung, insbesondere um eine Kobalt-Chrom-Molybdän-Legierung, handelt.

14. Hüftimplantatsystem (1) nach einem der vorhergehenden Ansprüche, **dadurch gekennzeichnet, dass** das Hüftimplantatsystem (1) ferner einen künstlichen Hüftgelenkeinsatz (30), vorzugsweise aufweisend oder bestehend aus Polyethylen, vorzugsweise ultrahochmolekularem Polyethylen, und optional einem Antioxidans, und/oder einen künstlichen Hüftgelenkkopf (40), insbesondere mit oder ohne künstlichem Hüftschaft (60), aufweist.

## Claims

1. A hip implant system (1), comprising
- an artificial acetabular cup (10) and
- an artificial acetabular liner (20) comprising or consisting of a metal or an alloy,
wherein the artificial acetabular liner (20) is coated at least in sections with a ceramic coating (50), **characterized in that** the ceramic coating comprises chromium nitride, chromium carbonitride and zirconium nitride.

2. The hip implant system (1) as claimed in claim 1, **characterized in that** only an outer face (22), in particular only a convex, conical or frustoconical outer face, of the artificial acetabular liner (20) or only an inner face (24), in particular only a concave, conical or frustoconical inner face, of the artificial acetabular liner (20) is coated at least in sections with the ceramic coating (50).

3. The hip implant system (1) as claimed in claim 1, **characterized in that** the artificial acetabular liner (20) is coated fully with the ceramic coating (50) .

4. The hip implant system (1) as claimed in one of the preceding claims, **characterized in that** the ceramic coating (50) is constructed in a plurality of layers.

5. The hip implant system (1) as claimed in one of the preceding claims, **characterized in that** the ceramic coating (50) comprises at least one chromium nitride layer, at least one chromium carbonitride layer, at least one chromium zirconium nitride layer and at least one zirconium nitride layer.

6. The hip implant system (1) as claimed in one of the preceding claims, **characterized in that** the ceramic coating (50) comprises three chromium nitride layers, two chromium carbonitride layers, one chromium zirconium nitride layer and one zirconium nitride layer, or consists of the aforementioned layers.

7. The hip implant system (1) as claimed in one of the preceding claims, **characterized in that** the ceramic coating (50) is formed as a multilayer layer system in which a first chromium nitride layer (15a) is covered by a first chromium carbonitride layer (15b), the first chromium carbonitride layer (15b) is covered by a second chromium nitride layer (15c), the second chromium nitride layer (15c) is covered by a second chromium carbonitride layer (15d), the second chromium carbonitride layer (15d) is covered by a third chromium nitride layer (15e), the third chromium nitride layer (15e) is covered by a chromium zirconium nitride layer (15f) and the chromium zirconium nitride layer (15f) is covered by a zirconium nitride layer (15g).

8. The hip implant system (1) as claimed in claim 6 or 7, **characterized in that** the zirconium nitride layer (15g) forms an outer cover layer of the ceramic coating.

9. The hip implant system (1) as claimed in one of the preceding claims, in particular as claimed in claim 6 or 7, **characterized in that** the ceramic coating (50), in particular one of the chromium nitride layers or the first chromium nitride layer (15a), is formed directly on the artificial acetabular liner (20).

10. The hip implant system (1) as claimed in one of the preceding claims, **characterized in that** the ceramic coating (50) has a thickness of from 0.5 µm to 10 µm, in particular from 1.5 µm to 7 µm, preferably from 2.5 µm to 5 µm.

11. The hip implant system (1) as claimed in one of the preceding claims, **characterized in that**
- an inner face of the artificial acetabular liner (20) has a surface roughness of ≤ 0.5 µm, preferably ≤ 0.05 µm, and/or
- an outer face of the artificial acetabular liner (20) has a surface roughness of ≤ 1 µm, preferably ≤ 0.6 µm, and/or
- the artificial acetabular liner (20) has a wall thickness of from 2 mm to 3 mm, and/or
- the artificial acetabular liner (20) has a cone angle θ of from 15° to 25°, in particular from 16° to 21°.

12. The hip implant system (1) as claimed in one of the preceding claims, **characterized in that** the artificial acetabular liner (20) and the artificial acetabular cup (10) in a joined state are connected to one another with a friction fit, in particular conically clamped to one another.

13. The hip implant system (1) as claimed in one of the preceding claims, **characterized in that** the alloy is an alloy containing cobalt and/or chromium, in particular a cobalt-chromium-molybdenum alloy.

14. The hip implant system (1) as claimed in one of the preceding claims, **characterized in that** the hip implant system (1) furthermore comprises an artificial femoral liner (30), preferably comprising or consisting of polyethylene, preferably ultrahigh molecular weight polyethylene, and optionally an antioxidant, and/or an artificial femoral head (40), in particular with or without an artificial femoral stem (60).

## Revendications

1. Système (1) de prothèse de hanche, présentant
- un cotyle (10) artificiel et
- un insert (20) artificiel de cotyle, présentant ou constitué par un métal ou un alliage,
l'insert (20) artificiel de cotyle étant revêtu au moins par endroits par un revêtement en céramique (50), **caractérisé en ce que** le revêtement en céramique présente du nitrure de chrome, du carbonitrure de chrome et du nitrure de zirconium.

2. Système (1) de prothèse de hanche selon la revendication 1, **caractérisé en ce que** seule une surface externe (22), en particulier seule une surface externe convexe, conique ou tronconique, de l'insert (20) artificiel de cotyle ou seule une surface interne (24), en particulier seule une surface interne concave, conique ou tronconique, de l'insert (20) artificiel de cotyle est revêtue au moins par endroits par le revêtement en céramique (50).

3. Système (1) de prothèse de hanche selon la revendication 1, **caractérisé en ce que** l'insert (20) artificiel de cotyle est complètement revêtu par le revêtement en céramique (50).

4. Système (1) de prothèse de hanche selon l'une des revendications précédentes, **caractérisé en ce que** le revêtement en céramique (50) est constitué de manière multicouche.

5. Système (1) de prothèse de hanche selon l'une des revendications précédentes, **caractérisé en ce que** le revêtement en céramique (50) présente au moins une couche de nitrure de chrome, au moins une couche de carbonitrure de chrome, au moins une couche de nitrure de chrome et de zirconium et au moins une couche de nitrure de zirconium.

6. Système (1) de prothèse de hanche selon l'une des revendications précédentes, **caractérisé en ce que** le revêtement en céramique (50) présente trois couches de nitrure de chrome, deux couches de carbonitrure de chrome, une couche de nitrure de chrome et de zirconium et une couche de nitrure de zirconium ou est constitué par les couches susmentionnées.

7. Système (1) de prothèse de hanche selon l'une des revendications précédentes, **caractérisé en ce que** le revêtement en céramique (50) est réalisé sous forme de système stratifié multicouche, dans lequel une première couche de nitrure de chrome (15a) est revêtue par une première couche de carbonitrure de chrome (15b), la première couche de carbonitrure de chrome (15b) est revêtue par une deuxième couche de nitrure de chrome (15c), la deuxième couche de nitrure de chrome (15c) est revêtue par une deuxième couche de carbonitrure de chrome (15d), la deuxième couche de carbonitrure de chrome (15d) est revêtue par une troisième couche de nitrure de chrome (15e), la troisième couche de nitrure de chrome (15e) est revêtue par une couche de nitrure de chrome et de zirconium (15f) et la couche de nitrure de chrome et de zirconium (15f) est revêtue par une couche de nitrure de zirconium (15g).

8. Système (1) de prothèse de hanche selon la revendication 6 ou 7, **caractérisé en ce que** la couche de nitrure de zirconium (15g) forme une couche de recouvrement externe du revêtement en céramique.

9. Système (1) de prothèse de hanche selon l'une des revendications précédentes, en particulier selon la revendication 6 ou 7, **caractérisé en ce que** le revêtement en céramique (50), en particulier l'une des couches de nitrure de chrome ou la première couche de nitrure de chrome (15a), est formée directement sur l'insert (20) artificiel de cotyle.

10. Système (1) de prothèse de hanche selon l'une des revendications précédentes, **caractérisé en ce que** le revêtement en céramique (50) présente une épaisseur de 0,5 µm à 10 µm, en particulier de 1,5 µm à 7 µm, de préférence de 2,5 µm à 5 µm.

11. Système (1) de prothèse de hanche selon l'une des revendications précédentes, **caractérisé en ce que**
- une surface interne de l'insert (20) artificiel de cotyle présente une rugosité de surface ≤ 0,5 µm, de préférence ≤ 0,05 µm et/ou
- une surface externe de l'insert (20) artificiel de cotyle présente une rugosité de surface ≤ 1 µm, de préférence ≤ 0,6 µm et/ou
- l'insert (20) artificiel de cotyle présente une épaisseur de paroi de 2 mm à 3 mm et/ou
- l'insert (20) artificiel de cotyle présente un angle conique θ de 15° à 25°, en particulier de 16° à 21°.

12. Système (1) de prothèse de hanche selon l'une des revendications précédentes, **caractérisé en ce que** l'insert (20) artificiel de cotyle et le cotyle (10) artificiel, dans un état assemblé, sont reliés l'un à l'autre par friction, en particulier reliés l'un à l'autre coniquement par serrage.

13. Système (1) de prothèse de hanche selon l'une des revendications précédentes, **caractérisé en ce qu'**il s'agit, pour l'alliage, d'un alliage contenant du cobalt et/ou du chrome, en particulier d'un alliage de cobaltchrome-molybdène.

14. Système (1) de prothèse de hanche selon l'une des revendications précédentes, **caractérisé en ce que** le système (1) de prothèse de hanche présente en outre une cupule (30) artificielle, de préférence, présentant ou constituée par du polyéthylène, de préférence, du polyéthylène à poids moléculaire ultra-élevé, et éventuellement un antioxydant et/ou une tête fémorale (40) artificielle, en particulier avec ou sans tige fémorale (60) artificielle.
